# EUROPEAN PATENT APPLICATION

(11) **EP 2 887 067 A1**
(43) Date of publication of application: **24.06.2015**
(21) Application number: 13198450.2
(22) Date of filing: 19.12.2013
(51) Int. Cl.: G01N 33/50, G01N 33/68, G01N 33/74

(54) **Quantification of glucocorticoids in fish scales as biomarkers for chronic stress**

(71) Applicant: Universiteit Gent, 9000 Gent (BE); Instituut Voor Landbouw- En Visserijonderzoek, 9820 Merelbeke (BE)
(72) Inventor: Aerts, Johan, 2970 Schilde (BE); De Saeger, Sarah, 9300 Aalst (BE)
(74) Representative: Jacobs, Philippe

(57) **Abstract**

The present invention relates to determining the stress level experienced by fish which can be used in assessing the welfare status of fish. More in particular, the present invention relates to the usage of scales sampled from fish to quantify glucocorticoid levels in said scales. The levels of said glucocorticoids -such as cortisol or corticosterone- build up over time in scales and reflect long lasting and stressful conditions which the fish have undergone during their lives and which have affect their level of stress. Hence, the present invention discloses in vitro methods to quantify the level of chronic stress in fish using scales as a logistic feasible, and non-invasive matrix for accurate and precise quantification of said stress hormones.

## Description

### Technical field of invention

The present invention relates to determining the stress level experienced by fish which can be used in assessing the welfare status of fish. More in particular, the present invention relates to the usage of scales sampled from fish to quantify glucocorticoid levels in said scales. The levels of said glucocorticoids - such as cortisol or corticosterone - build up over time in scales and reflect long lasting and stressful conditions which the fish have experienced during their lives and which have affected their level of stress. Hence, the present invention discloses in vitro methods to quantify the level of chronic stress in fish using scales as a logistic feasible, and non-invasive matrix for accurate and precise quantification of said stress hormones.

### Background art

Scientifically validated methods to assess animal welfare are needed to accommodate an increasing societal demand for more sustainable and animal-friendly protein production. In aquaculture, major stressors are stocking density, water quality (inadequate temperature, hypoxic conditions, high ammonia and carbon dioxide concentrations), pollutants and confinement. Due to an often large heterogeneity in growth rate of cultured fish, frequent size grading is necessary causing significant handling stress¹. All vertebrates, when faced with a stressful stimulus, respond by activation of the endocrine stress axis (hypothalamus-pituitary-interrenal (HPI) axis in fish, adrenal (HPA) in 'higher' vertebrates), and release cortisol²⁻³ or corticosterone⁴⁻⁵ in the bloodstream. The glucocorticoid then elicits a suite of physiological and behavioral changes known as the stress response⁶⁻⁷. Glucocorticoids help an organism to cope with the altered situation⁸⁻¹⁰. The adaptive value of short-term elevation of glucocorticoids is well recognized. Far less is known about chronically elevated levels and assumed detrimental consequences for growth, reproduction, immunity and body condition⁶⁻⁷.
Blood levels of glucocorticoids are widely used to evaluate the effects of various stressors¹¹⁻¹² However, the mere act of blood sampling causes stress to the animal and is sometimes impossible. In addition, short-term handling of fish under anesthesia may avoid activation of the HPI axis¹³⁻¹⁴. Following slaughter, adequate blood samples are not available.
Alternatively, cortisol can be determined non-invasively in matrices like water¹⁶⁻¹⁷ or feces¹⁸⁻¹⁹. However, these matrices are not feasible to sample in wild life nor in commercial aquaculture settings where open cages, flow-through or recirculation systems prevail. Additionally, cortisol concentrations in feces reflect several interacting processes including conjugation in the liver, passage in the gastrointestinal tract and bacterial breakdown, all confounding their interpretation²⁰. Analyzing glucocorticoid levels in the blood, feces or water at a fixed time point furthermore has the major disadvantage of only creating a snap-shot, not a reflection of the stress experienced by the animal over a longer time period. A similar reasoning applies when considering measuring cortisol levels in skin mucus as has been disclosed by Bertotto et al.²¹. The latter authors further consider gut content, lateral muscle and caudal fin as alternative matrices for cortisol measurement but this again requires an invasive sampling method often via killing the animal and are matrices unsuited for chronic stress related research. Hence, a feasible glucocorticoid-containing matrix that adequately assesses long-term exposure to stress in a non-invasive manner is urgently needed.
A biomarker that adequately assesses long-term exposure to stress is needed. Hitherto, the majority of studies addressed cortisol, and only scarce data are available on cortisone as well as other related glucocorticoids such as 11-deoxycortisol and their significance in chronic stress research.
Fish record the principal life events related to external physicochemical conditions and store their physiological history in their calcified tissues²². Fish continue to grow during their life and the continuously growing scales, spines, soft fin rays, and otoliths are hard tissues storing this 'memory'. Scales are dermal bone plates in teleost skin and display growth dependent concentric rings or annuli²³⁻²⁷. Scales have not been considered before as a feasible matrix for determining glucocorticoid levels in a non-invasive manner in order to measure chronic stress in animals.

### Brief description of figures

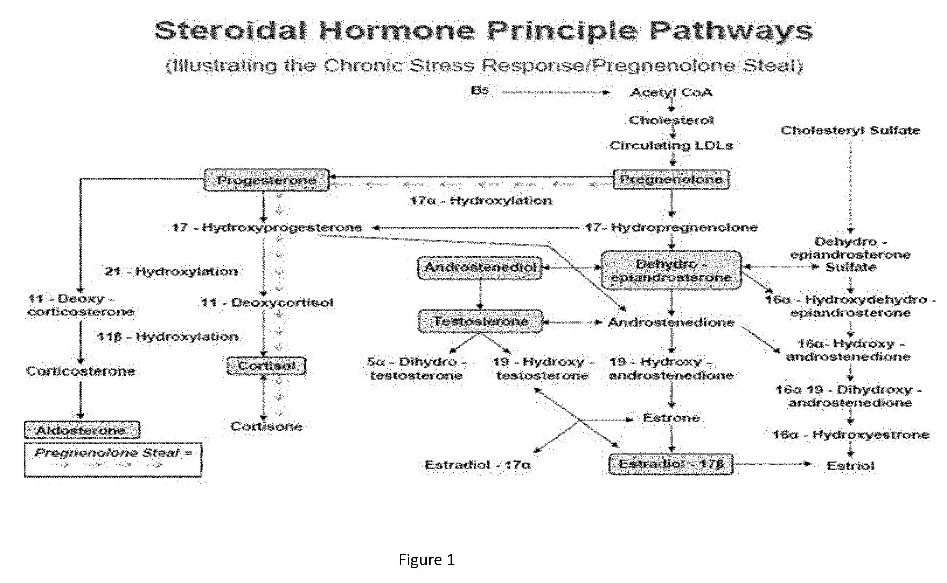

Figure 1: Steroidal hormone principle pathways and analytes involved in the chronic stress respons.²⁸

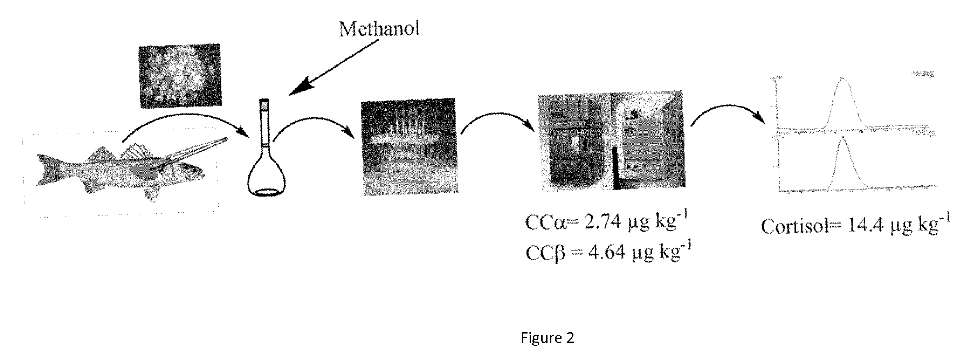

Figure 2: Schematic overview of the method.
Figure 3: Optimal amounts of matrix for scale, spine and soft fin ray for validation.
Figure 4: Optimization of extraction time and repeats for cortisol in scale, spine and soft fin ray.
Figure 5: Calibration curve in diluent and in matrix for cortisol in fish scales.

### Description of invention

The present invention relates to the finding that the glucocorticoid profile in fish is captured in scales, and that glucocorticoid levels in these dermal bone plates reflect a time-integrated sample of the degree of stress the fish experienced over time. Scales are indeed considered as an ideal matrix for chronic stress monitoring in fish as scales are easily sampled in a non-invasive manner and as scales allow convenient sample preparation for further quantification of glucocorticoid levels in said scales. The present invention thus relates to accurate, precise and robust methods for quantification of glucocorticoids such as cortisol in fish scales to assess exposure of the fish to stress during an extended period of time.
More specifically, the present invention relates to an in vitro method to quantify the level of chronic stress in a fish comprising:
- obtaining at least one scale from said fish,
- extracting glucocorticoids from said scales,
- purifying the glucocorticoids extracted from said scales,
- quantifying said purified glucocorticoids, and
- correlating said quantified glucocorticoids with a level of chronic stress

The term 'glucocorticoids' means cortisol when scales are taken from a fish belonging to the infraclass Teleostei, and/or, means corticosterone when scales are taken from a fish belonging to the class Chondrichtyes, and/or means - when the detection of possible extra-interrenal pathways is additionally required - precursors of cortisol such as 17α-hydroxyprogesterone and/or 11-deoxycortisol and/or precursors of corticosterone such as 11-deoxycorticosterone, and/or means metabolites of cortisol such as cortisone, tetrahydrocortisol and/or tetrahydrocortisone and/or metabolites of corticosterone as these metabolites may influence the glucocorticoid levels in the scales so that their detection is additionally required.

The chemical structure of the above indicated glucocorticoids is the following²⁹:
- Cortisol or 11β,17α,21-trihydroxypregn-4-ene-3,20-dione: 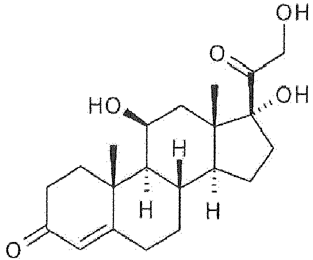
- Corticosterone or 11β,21-Dihydroxy-4-pregnene-3,20-dione: 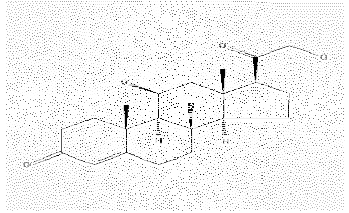
- 17α-Hydroxyprogesterone or 17α-Hydroxypregn-4-ene-3,20-dione: 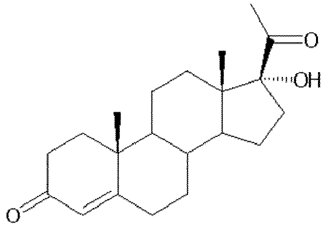
- 11-Deoxycortisol or 17α, 21-Dihydroxypregn-4-ene-3, 20- dione: 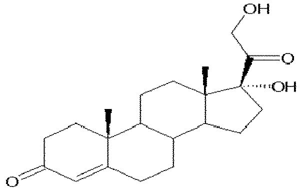
- 11-Deoxycorticosterone or 21-hydroxypregn-4-ene-3,20-dione 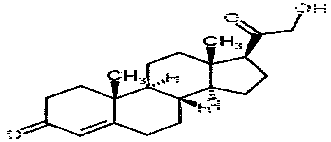
- Cortisone or 17α,21-Dihydroxy-pregn-4-een-3,11,20-trion: 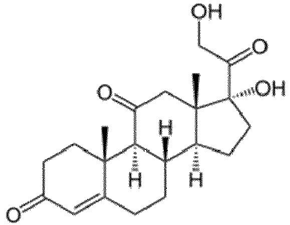
- Tetrahydrocortisol or 3α,11ß,17α,21-tetrahydroxy-5ß-pregnaan-20-on: 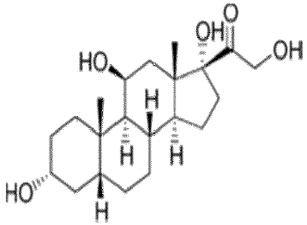
- Tetrahydrocortisone or 3α,17α,21-trihydroxy-5f3-pregnaan-11. 20-dion:

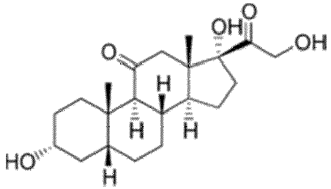

Hence, the present invention relates to a method as described here wherein said glucocorticoids are cortisol, 17α-hydroxyprogesterone, 11-deoxycortisol, corticosterone, 11-deoxycorticosterone cortisone, tetrahydrocortisol and/or tetrahydrocortisone. As the classical parameter considered to reflect the stress response in teleost fish, cortisol is released into the bloodstream making incorporation in calcified structures possible and hereby a prime target analyte for chronic stress research. Its precursors 17α-hydroxyprogesteron and 11-deoxycortisol were included in the method to unravel the existence of possible extra-interrenal pathways. As the existence of extra-interrenal pathways in fish are at present not known, but as there are indications of extra-adrenal pathways in humans³⁰, both analytes were incorporated in the method for future research purposes. The existence of such a pathway could have a profound effect on our understanding of the HPI-axis. The metabolites cortisone, tetrahydrocortisol and tetrahydrocortisone were also included in the method as exogenous glucocorticoids in the water (coming from other fish, antropogenic activities, etc..) may act as an endocrine disruptor possibly influencing the glucocorticoid profile in the scales.

The term 'fish' means in essence any gill-bearing, craniate aquatic animal that has scales but specifically refers to fish belonging to the taxonomic class of the Chondrichtyes (cartilaginous fish) or to the taxonomic infraclass of Teleostei (bony fish).

The present invention further specifically relates to a method as described above, wherein said fish belong to the infraclass of Teleostei.

More specifically, the present invention relates to a method as described above wherein said fish belonging to the infraclass of Teleostei are fish belonging to the family Moronidae (e.g. European Sea bass), the family Sparidae (e.g. Sea breams), the family Soleidae (e.g. Solea solea and Solea senegalensis), the family Salmonidae (e.g. salmon), the so-called Kingfish (e.g. Yellow tail Kingfish), the family Scombridae (e.g. Tuna), the family Gadidae (e.g. Cod), the family Cyprinidae (e.g. common carp), and cichlid fish (e.g. Mozambique tilapia).

The term 'scale' means dermal bone plates typically found in fish skin which display growth dependent concentric rings or annuli.

The term 'chronic stress' in fish means stress build up over time and reflecting long lasting and stressful conditions due to handling, poor water quality, stocking density, predation and several other antropogenic factors such as windmills, boat traffic, touching, etc. Chronic stress is thus a long lasting condition from which fish cannot fully recover. The direct effects of the stress factors, as well as changes occurring at endocrinological level, immune system or function level can be responsible for (pre)pathological consequences which reduce welfare of said fish.

The term 'obtaining scales from a fish' means removing at least one scale from the skin of said (living or dead) fish with for example fine tweezers. Said scales can be taken from any part of the body of said fish: i.e. dorsal, cranioventral, medioventral, caudal, left of right side, etc. To standardize the method of the present invention, scales were taken from the mediodorsoventral zone dorsally to the pectoral fin. This non-invasive manner of sample collection has no effect on the fish as the removed scales regrow within a short period of time.

As the weight of a single scale depends on the fish species as well as on the age of the fish, with smaller scales in juvenile fish, the number of scales needed depends on both of these factors. For this reason, the method of the present invention was standardized using a standardized amount of scales corresponding to for example 100 mg of scales, though tests point out that smaller amounts (such as 80, 60, 40, 20, 10, 5 mg, etc. of scales) can be used as well. The number of scales corresponding to 100 mg of scales is often about 40 to 80 scales, such as 60 scales, but it should be clear that the method of the present invention can be performed using 1, 2, 3, 4, 5, 6, 7, 8, 9, 10 or more scales, taking into account both factors as mentioned above.

The present invention thus also relates to a method as described above, wherein said obtaining at least one scale from said fish is obtaining between 40 and 80 scales from said fish.

The term 'extracting glucocorticoids from said scale' relates to any method to separate glucocorticoids from said scales. Typical -but non-limiting- extraction methods of the present invention involve the extraction of glucocorticoids out of an aqueous phase and into an organic phase. Extraction solvents that can for example be used are methanol, 2-propanol, acetonitrile, diethyl ether, etc. Methanol however is a preferred solvent.

Hence, the present invention relates to a method as described above, wherein said extracting glucocorticoids is undertaken by adding methanol as extraction solvent. An example of an extraction of the present invention is as follows: of the homogenized scale sample 0.1 g ± 0.001 g is weighed into a 10 ml test tube. Subsequently, 8 ml of methanol is added as extraction solvent and 10 µL of a cortisol-d₄ solution of 0.5 µg L⁻¹ is added as internal standard. The sample is vortex-mixed for 30 s, placed on an overhead shaker at 60 rpm for 1 h at room temperature and centrifuged for 10 min at 3500 g at 7 °C. All supernatant is taken, evaporated to dryness under nitrogen at 60 °C using a nitrogen evaporator, and reconstituted in 5 ml H₂O/MeOH (80:20; v/v) for subsequent Solid Phase extraction (SPE).

The term 'purifying the glucocorticoids extracted from said scale' relates to any method to render the glucocorticoids of the present invention pure or more pure as well as concentrated or more concentrated, and/or, to eliminate impurities as much as possible.

More in particular, the present invention also relates to a method as described above, wherein said extraction with methanol is followed by a SPE. A non-limiting example of a purification step of the present invention is as follows: after conditioning a C18 SPE column with 3 ml of methanol followed by 3 ml of ultrapure water, a sample as obtained above is loaded. The column is washed with 4.5 ml H₂O/MeOH (65:35; v/v) and retained compounds are eluted with 2.5 ml H₂O/MeOH (20:80; v/v) into a 10 ml test tube and evaporated to dryness under nitrogen at 60 °C using a nitrogen evaporator. The sample is finally reconstituted in 100 µL H₂O/MeOH (80:20; v/v) in a vial and analyzed by means of ultra-performance liquid chromatography coupled to tandem mass spectrometry (UPLC-MS/MS).

The term 'quantifying said purified glucocorticoid' refers to any method to determine the amount of glucocorticoids obtained after extraction/purification as described above.

More specifically, the present invention relates to a method as described above, wherein said quantifying of extracted and purified glucocorticoids is undertaken by liquid chromatography coupled to tandem mass spectrometry ((U)HPLC-MS/MS)³¹⁻³², high performance liquid chromatography coupled to ultraviolet detection (HPLC-UV) and Diode Array Detection (HPLC-DAD)³³³⁴, or fluorescence detection (HPLC-FL)³⁵⁻³⁶, gas chromatography (GC)³⁷⁻³⁸, an (radio)(enzymatic)immunoassay (RIA)³⁹⁻⁴⁰ (EIA)⁴¹⁻⁴² or a sensor based technique⁴³⁻⁴⁴.

The present invention also relates to a method as described above, wherein said liquid chromatography coupled to tandem mass spectrometry is ultra-performance liquid tandem mass spectrometry.

More specifically, the present invention relates to a method as described above, wherein said immunoassay is a radio immunoassay or an enzyme immunoassay.

The term 'correlating said quantified glucocorticoids with a level of chronic stress' refers to the fact that stressed teleost fish produce cortisol which is released into the bloodstream. When this stress load is prolonged in time, cortisol circulates during this prolonged period in the blood. Taking into account the structure of a scale, a certain percentage of this bioactive and free circulating cortisol is incorporated into the scale. As scales grow in time, this gives the opportunity to quantify the cortisol and thus the stress load in time. Knowing this, scales of fish exposed to a varying stress load can be analyzed for glucocorticoids. For example, fish which are exposed to a minor stress load (i.e. confinement into a reduced volume (such as 25% of the 'original' volume) for 30 min every 2nd day) have an average cortisol amount of 1.70µg kg⁻¹, while this is 3.44µg kg⁻¹ in highly stressed fish (i.e. confinement into 10% volume for 30 min, + chasing 5 min every 2nd day, + air exposure for 1 min once per week).

The present thus relates in essence to the usage of fish scales sampled from a fish to determine or quantify the level of chronic stress of said fish.

The present invention relates to the usage as described above wherein said quantification/determination is undertaken by a method according to the methods described above.

The present invention will now be illustrated by the following non-limiting examples.

### Example 1: Development and validation of an UPLC-MS/MS method for quantifying glucocorticoids in scales of European sea bass (Dicentrarchus labrax)

### METHODS AND MATERIALS

### Instrumentation, materials, and reagents

Chromatographic analysis was performed on an Acquity UPLC-MS/MS Premier XE using an Acquity Ultra Performance LC BEH C18 (1.7 µm; 2.1 x 100 mm) column (Waters, Milford, USA). Samples were evaporated to dryness with a Turbovap™ nitrogen evaporator (Biotage, Sweden). Grace Pure™ SPE C18-Max (500 mg, 6 ml) columns for solid-phase extraction (SPE) were obtained from Grace Davison Discovery Sciences (Lokeren, Belgium). High-performance liquid chromatography (HPLC)-gradient grade methanol (Hipersolv Chromanorm) as extraction solvent was obtained from VWR International BVBA (Leuven, Belgium), while methanol absolute LC-MS as well as formic acid ULC-MS grade from Biosolve BV (Valkenswaard, The Netherlands) and ultrapure water of a Milli-Q gradient Q-Gard 2 from Millipore (Billerica, USA) were used as mobile phase solvents.

### Compounds, standards, and solutions

Only products with certificate of analysis were used. Cortisol, cortisone, 17α-hydroxyprogesteron, and 11-deoxycortisol were from Sigma-Aldrich (Diegem, Belgium). Tetrahydrocortisol and tetrahydrocortisone were from Sequoia Research Products Ltd (Pangbourne, United Kingdom). Cortisol-d₄ from CDN Isotopes (Pointe-Claire, Canada) was used as an internal standard.

Individual stock standard solutions of 1 mg mL⁻¹ of all compounds and internal standard were prepared in methanol and stored at 4 °C. Calibration standards, ranging from 0.005 mg L⁻¹ to 0.1 mg L⁻¹, were prepared by addition of 10 µl of a cortisol-d₄ solution of 0.5 µg L⁻¹ to 0.5 µl, 1 µl, 2.5 µl, 5 µl, and 10 µL of a standard solution of 1 µg L⁻¹ in 100 µL of H₂O/MeOH (80:20; v/v), respectively. As 100 mg of sample were used, this corresponded to a range from 5 µg kg⁻¹ to 100 µg kg⁻¹ in matrix.

### Sampling

All calcified structures were sampled from sacrificed European sea bass (*Dicentrarchus labrax)* using deeply anesthesised with 2-phenoxyerhanol and killed by spinal transection. All fish weighed around 100 g. Sixty scales from the left mediodorsoventral zone dorsally to the pectoral fin were removed from the skin with fine tweezers. Four dorsal spines and six soft fin rays from the caudal fin were retrieved using scissors. Two sagittal otoliths were taken from the opened skull. All samples were rinsed with ultrapure water and air dried on a paper tissue at room temperature.

### Sample preparation

Scales, spines, and soft fin rays were cut into fine pieces using scissors. Otoliths were ground in a mortar. Scissors and mortar were rinsed with ethanol followed by ultrapure water and dried with a paper tissue. Of the homogenized sample 0.1 g ± 0.001 g was weighed into a 10 ml test tube. Subsequently, 8 ml of methanol was added as extraction solvent and 10 µL of a cortisol-d₄ solution of 0.5 µg L⁻¹ was added as internal standard. The sample was vortex-mixed for 30 s, placed on an overhead shaker at 60 rpm for 1 h at room temperature and centrifuged for 10 min at 3500 g at 7 °C. All supernatant was taken, evaporated to dryness under nitrogen at 60 °C using a nitrogen evaporator, and reconstituted in 5 ml H₂O/MeOH (80:20; v/v). After conditioning a C18 SPE column with 3 ml of methanol followed by 3 ml of ultrapure water, sample was loaded. The column was washed with 4.5 ml H₂O/MeOH (65:35; v/v) and retained compounds were eluted with 2.5 ml H₂O/MeOH (20:80; v/v) into a 10 ml test tube and evaporated to dryness under nitrogen at 60 °C using a nitrogen evaporator. The sample was finally reconstituted in 100 µL H₂O/MeOH (80:20; v/v) in a vial and analyzed by means of UPLC-MS/MS.

### LC-MS/MS analysis

Glucocorticoids were separated using a gradient elution of mobile phases A and B. Mobile phase A was a mixture of ultrapure water with 0.1 % formic acid, while mobile phase B was a mixture of methanol with 0.1 % formic acid. Initially, gradient elution started at 20 % (v/v) of mobile phase B. Subsequently, mobile phase B was increased to 56 % at 1.5 min, to 63 % at 6.5 min, to 99.1 % at 7.5 min after which it was kept at 99.1 % to 8 min, and finally decreased to 20 % at 9 min and kept in this way to 10 min. The flow rate was kept constant at 0.4 ml min⁻¹, resulting in a 10 min running time. Samples were cooled at 7 °C in the autosampler. The injection volume was set at 40 µl, while the column temperature was maintained at 30 °C.

Chromatographic analysis was performed on a mass spectrometer used in the multiple reactions monitoring (MRM) mode in order to achieve optimal sensitivity and selectivity. For every target analyte, two precursor fragment ion transitions were determined. Instrumental parameters were optimized by the direct infusion of 10 µg L⁻¹ standard solution in methanol + 0.1 % formic acid at a flow rate of 10 µL min⁻¹. The use of two precursor fragment ion transitions allowed the determination of the ratio between both transitions, which was used together with the relative retention time for the identification and confirmation of the identity of each analyte according the requirements of the Commission Decision No. 2002/657/EC⁴⁵. The mass spectrometer was used in positive electrospray ionization mode (ESI⁺). All compounds were analyzed as their proton adducts [M+H]⁺. The MS detector settings were set at the following values: a source temperature of 120 °C, a de-solvation temperature of 300 °C at a gas flow of 800 L h⁻¹, a cone gas flow of 50 L h⁻¹, and a capillary voltage of 3 kV. Argon was used as collision gas at a pressure of 1.11. 10⁻² mbar. The optimized UPLC-MS/MS conditions with indication of retention time, precursor ion, product ions, cone voltage, and collision energy for all compounds are given in Table 1.

**Table 1. Optimized UPLC-MS/MS conditions per compound**

| Compound | Retention time (min) | Precursor ion (m/z) | Fragment ion - quantification* and qualification trace (m/z) | Cone voltage (V) | Collision energy (eV) |
|---|---|---|---|---|---|
| Cortisone | 2.88 | 361.2 | 121.00* | 45 | 37 |
| | | | 163.00 | | 24 |
| Cortisol-d₄ | 3.14 | 367.3 | 121.00* | 35 | 20 |
| | | | 331.00 | | 18 |
| Cortisol | 3.15 | 363.2 | 121.10* | 35 | 20 |
| | | | 327.30 | | 18 |
| 11-Deoxycortisol | 3.95 | 347.3 | 97.10* | 40 | 25 |
| | | | 109.05 | | 30 |
| Tetrahydrocortisol | 4.26 | 367.1 | 313.20* | 20 | 10 |
| | | | 331.30 | | 10 |
| Tetrahydrocortisone | 4.49 | 365.5 | 329.44* | 30 | 15 |
| | | | 347.46 | | 12 |
| 17α-Hydroxyprogesteron | 5.66 | 331.3 | 97.00* | 35 | 25 |
| | | | 109.10 | | 25 |

Data analysis was performed using Quanlynx software from Waters; analysis results were reported as the value (µg kg⁻¹) ± the expanded measurement uncertainty (µg kg⁻¹) with a coverage factor (k) of 2 (95 % confidentiality interval).

### Validation

Validation samples for every calcified structure were made by pooling of scales, spines, soft fin rays, and otoliths from 51, 51, 57, and 118 fish, respectively, reared under similar conditions. No certified reference material, interlaboratory comparison tests or any other validated methods for the above mentioned analyte/matrix combinations exist, and therefore validation was done using standard addition to validation samples. For every calcified structure five concentration levels, ranging from 1 to 50 µg kg⁻¹, were tested in five-fold and this was repeated on four different days within a period of one month under intra-reproducibility conditions, i.e. by two persons using different solutions and one UPLC-MS/MS system. All validation experiments were carried out by authorized personnel in controlled environment with calibrated equipment and controlled solutions according to the requirements of the standard EN ISO/IEC 17025:2005⁴⁶. Analysis was done using standardized sequences consisting of different calibration standards, blanks, negative and positive controls (all in diluent). Results for every compound were evaluated by assessing the (relative) retention time and relative ion intensities of the compound and fragments. All validation parameters were determined and evaluated according the requirements of the Commission Decision No. 2002/657/EC: trueness, precision, working range, linearity, decision limit (CCα), detection capability (CCβ), sensitivity, and selectivity.
The apparent recovery as well as precision (repeatability and intra-laboratory reproducibility) for all compounds were determined under intra-reproducibility conditions resulting in 20 analyses for every concentration level and a total of 100 analyses per compound and matrix. Due to the low concentration levels, a Dixon's outlier test⁵⁹ as well as a Grubbs'⁶⁰ test were performed to detect possible outliers. The decision limit (CCα) was calculated as the intercept of the calibration curve plus 2.33 times the standard deviation on the intra-laboratory reproducibility (α = 1 %), while the detection capability (CCβ) was calculated as the concentration of CCα plus 1.64 times the standard deviation on the intra-laboratory reproducibility (β = 5 %). The expanded measurement uncertainty was based on the validation data for trueness and intra-laboratory reproducibility and determined using two different approaches, by linear summation and by quadratic summation or Nordtest method. In addition the robustness of the method and stability of the analytes in diluent as well as in matrix were monitored during method development. Finally the expanded measurement uncertainty was determined by linear summation as well as by quadratic summation or Nordtest method⁴⁷⁻⁵² (there is no consensus in the literature on a preferred method).

### RESULTS

An UPLC-MS/MS quantification method for cortisol, its precursors 17α-hydroxyprogesterone and 11-deoxycortisol, cortisone and key metabolites tetrahydrocortisol and tetrahydrocortisone in fish scales was developed in an EN ISO/IEC 17025:2005 accredited environment and validated according the requirements of the Commission Decision No. 2002/657/EC. As the classical parameter considered to reflect the stress response in most fish, cortisol is released into the bloodstream making incorporation in calcified structures possible and hereby a target analyte for chronic stress research. Its precursors 17α-hydroxyprogesteron and 11-deoxycortisol were included in the analyses to cover detection of possible extra-interrenal pathways. The metabolites tetrahydrocortisol and tetrahydrocortisone were also included as exogenous glucocorticoids (in the water) may act as endocrine disruptor and influence the glucocorticoid profile of the calcified structures studied. The method was tested and validated for spine, soft fin ray, and otolith as well. The convenience of scale sampling is addressed.

### Method development

### Sampling and sample preparation

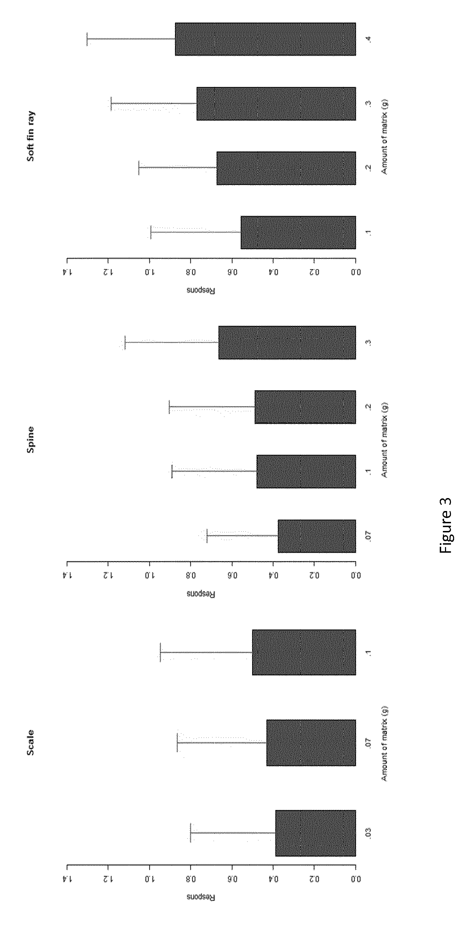

For each calcified structure the location for sampling was determined. Scales are mesodermal skeletal elements in postmetamorphosis fish. No differences were found in cortisol content in scales taken from the various sides (e.g. dorsal, cranioventral, medioventral, or caudal) of the fish nor between scales taken from the left and right side. Scales for the lateral line should be avoided as they differ in structure. For ease and future research purposes (taking into account the applicability in aquaculture) scales were chosen from the mediodorsoventral zone dorsally to the pectoral fin. Spines in sea bass are found among others in the cranial part of the first dorsal fin (8 to 10 spines). Spines are not segmented, but fuse at the base and are therefore more rigid than soft fin rays. A higher concentration of glucocorticoids was found in spines at their base which requires better fine-tuning of cortisol incorporation over time in this tissue. In addition, all except the distal parts of the spine are covered by epithelium. No differences were found in cortisol content in soft fin rays taken from the dorsal, cranioventral, medioventral, and caudal fins. Taking into account the applicability in aquaculture soft fin rays were chosen from the caudal fin. Sagittal otoliths were chosen as they are the largest and easiest to remove.
In respect of an optimal combination of logistic feasibility and analytical performance, the optimal amount of matrix for of analysis was determined for each calcified structure by spiking different amounts of sample, i.e. different number of scales, spines and soft fin rays from the same fish, with 5 µg kg⁻¹ of the target analytes. Results for scale, spine and soft fin ray are presented in Figure 3. Taking into account the future applicability in aquaculture and wildlife 0.1 g of every calcified structure was chosen for validation.

### Figure 3. Optimal amounts of matrix for scale, spine and soft fin ray for validation

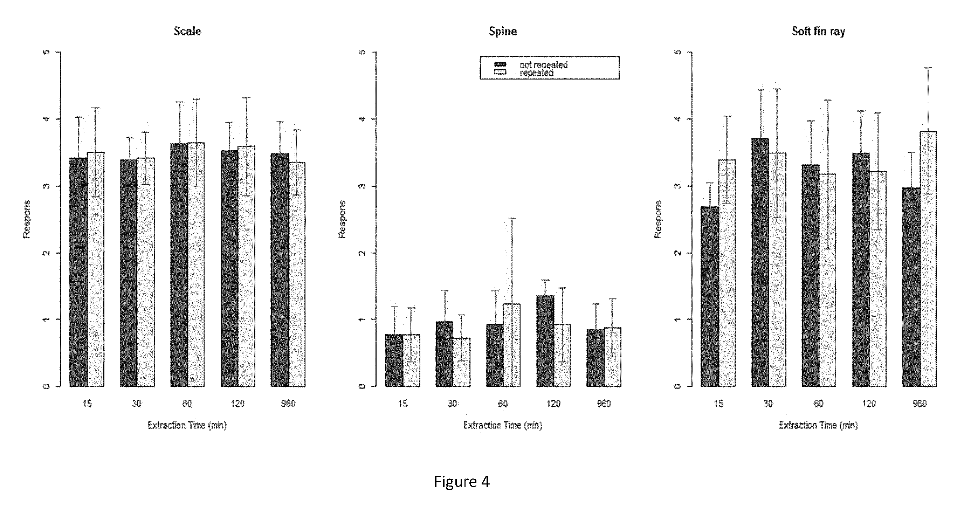

To remove mucus, known to contain endogenous⁵³, as well as exogenous glucocorticoids, intensive washing of all samples with ultrapure water was carried out.
Four solvents with different extraction efficiency were tested: methanol, 2-propanol, acetonitrile, and diethylether. Methanol gave the best results for the various glucocorticoids in scales as well as in spines and soft fin rays; in otoliths no clear difference between solvents was seen (data not shown).
Next, time-dependence of incubation with methanol (15, 60, 120 min and 16 h) as well as repeated extraction was tested. For cortisol in fish scales no improvement of extraction was seen after 60 min of incubation. No improvement of a second extraction was seen, nor of a combination of 15 min for 2 cycles (versus 1 cycle of 60 min). Results for scale, spine, and soft fin ray are presented in Figure 4.

### Figure 4. Optimization of extraction time and repeats for cortisol in scale, spine and soft fin ray

As a high throughput and cost effective method is needed, extraction in scales was standardized with 8 ml of methanol for 1 h on an overhead shaker at room temperature.

### SPE procedure

C18 phase SPE cartridges were successfully used to extract glucocorticoids from biological samples such as plasma⁵⁴, urine⁵⁵, feces⁵⁶, and therefore this column type was chosen to optimize the extraction and purification of the selected glucocorticoids from the calcified structures in this study.
Grace Pure™ SPE C18-Max (500 mg, 6 ml) columns are reversed phase sorbents with a polymerically bonded 17.1 % carbon load on a silica based support. The reversed phase extraction procedure was chosen since it binds moderately polar to non-polar compounds from a polar sample matrix. A bed size of 500 mg gives a sorbent retention capacity of 25 mg. Taking into account the predicted concentration range of glucocorticoids in scales as well as in the other calcified structures, this retention capacity will suffice to bind all analytes of interest. The conditioning, sample loading, washing, and elution steps for the SPE were optimized for solvent type and volume. The conditioning step with 3 ml of methanol to activate the sorbent ligands followed by 3 ml of ultrapure water to equilibrate the sorbent bed gave best results (average recovery of cortisol > 90 %). The minimal volume for both solvents was 2.5 ml, while tests using volumes over 3 ml did not improve purification. The sample loading step whereby the evaporated sample was resuspended in 5 ml H₂O/MeOH (80:20; v/v) gave the best results. Solvent combinations in which the proportion of ultrapure water and/or methanol were varied gave suboptimal results. Washing of the column with 4.5 ml H₂O/MeOH (65:35; v/v) gave the best results for glucocorticoid recovery. A higher concentration of methanol led to elution of glucocorticoids, while a lower concentration of methanol resulted in an increase of noise. The retained compounds were eluted with 2.5 ml H₂O/MeOH (20:80; v/v) into a 10 ml test tube. A lower elution volume or an increase of ultrapure water did not recover all glucocorticoids, while a higher elution volume or an increase of methanol was suboptimal regarding cost effectiveness and as it eluted more interfering compounds from the matrix, respectively. After evaporation, the sample was reconstituted in 100 µL H₂O/MeOH (80:20; v/v) in a vial and analyzed by means of LC-MS/MS. Reconstitution in 100 µL was chosen to have the possibility of a duplo injection. Solvent combinations in which the proportion ultrapure water to methanol were changed gave suboptimal results since the gradient for chromatographic analysis was optimized to start at H₂O/MeOH (80:20; v/v). Reconstitution in a concentration up to H₂O/MeOH (20:80; v/v) was possible but had negative effects on the chromatographic peak shapes.

### Chromatographic analysis

In a first step to optimize the chromatographic analysis using an Acquity Ultra Performance LC BEH C18 (1.7 µm; 2.1 x 100 mm) column, and taking into account the literature on hair⁵⁷⁻⁵⁸, all compounds as well as the internal standard were tuned using the mass spectrometer in positive (ESI⁺) and negative (ESI⁻) electrospray ionization mode. Results obtained in ESI⁻ gave small peaks but less noise while ESI⁺ gave overall better results and was chosen for further testing.
In a second step, different gradient systems using ultrapure water and nonpolar solvents like methanol and acetonitrile with 0.1 % of formic acid were tested. At first gradients using ultrapure water and acetonitrile were tested: a higher starting concentration of acetonitrile gave a shorter retention time and only separation of cortisol, cortisone and 17α-hydroxyprogesteron was seen; running a gradient increasing slowly the acetonitrile concentration resulted in loss of the 17α-hydroxyprogesteron peak as well as a runtime of 12 min making acetonitrile unsuitable as mobile phase. Subsequently gradients using ultrapure water and methanol were tested: after running different gradients ranging from a slow to a very quick increase of the methanol concentration all compounds were fully separated and variable concentrations of different additives were tested. The use of 0.5 % acetic acid gave better results than 2 mM ammonium formiate at pH 3.0 or ammonium acetate at pH 5.0, but poorer than 0.1 % formic acid. Subsequently the concentration of formic acid was optimized and 0.1 % formic acid was chosen ; a lower concentration of formic acid resulted in unstable results due to the carry-over from methods using other additives. Finally the column temperature and flow rate were optimized at 30 °C and 0.4 ml min⁻¹, respectively, to have a total run time of 10 min.
In a last step the MS/MS method was optimized: dwell times were increased for an optimal sensitivity to secure that the points across the peak for every ion of all compounds consisted of at least 20 data points and MS frame windows were optimized accordingly.

### Validation

Concentration values of glucocorticoids in scales (or any other calcified structure) are not available in the published literature, which made us opt for an initial working range from 1 µg kg⁻¹ to 50 µg kg⁻¹.
The apparent recovery for cortisol in fish scales below, at and above CCβ level ranged from 88.14 % to 98.85 %, all within the 80 % to 110 % performance criterion according to the requirements of the Commission Decision No. 2002/657/EC. Despite the fact that the homogenization of the pooled validation sample was not optimal (i.e. it was not a homogenous powder, but rather a pool of very fine fragments), the repeatability for cortisol in fish scales ranged from 12.36 % to 17.15 %. The intra-laboratory reproducibility ranged from 14.63 % to 20.02 %. The results for trueness, precision and measurement uncertainty for the analyzed compounds in fish scales are presented in Table 2.

**Table 2: Values for trueness (apparent recovery AR, percent), coefficient of variation values (CV, percent) for precision (repeatability CVᵣ and intra-laboratory reproducibility CV_{R}) and expanded measurement uncertainty (U, percent) for all compounds in fish scale**

| Compound | Level (µg kg⁻¹⁾ | AR (%) | CVᵣ (%) | CV_{R} (%) | U¹(k=2) (%) | U¹(k=3) (%) | U²(k=2) (%) |
|---|---|---|---|---|---|---|---|
| Cortisol | 1 | 98.85 | 17.15 | 20.02 | 41.19 | 61.21 | 66.16 |
| | 5 | 88.14 | 12.36 | 15.01 | 41.88 | 56.89 | 58.08 |
| | 10 | 98.20 | 16.21 | 18.05 | 37.90 | 55.95 | 61.44 |
| | 25 | 90.97 | 13.81 | 14.85 | 38.73 | 53.58 | 56.08 |
| | 50 | 91.16 | 13.47 | 14.63 | 38.10 | 52.73 | 55.57 |

| **Precursors** | | | | | | | |
|---|---|---|---|---|---|---|---|
| 17α-Hydroxyprogesteron | 1 | 114.00 | 27.83 | 32.08 | 78.16 | 110.24 | 106.46 |
| | 5 | 104.44 | 33.09 | 43.74 | 91.92 | 135.66 | 31.31 |
| | 10 | 123.75 | 13.09 | 21.98 | 67.71 | 89.69 | 91.08 |
| | 25 | 135.64 | 6.64 | 16.23 | 68.10 | 84.33 | 96.06 |
| | 50 | 141.34 | 7.79 | 18.38 | 78.10 | 96.48 | 109.63 |
| 11-Deoxycortisol | 1 | -³ | -³ | -³ | -³ | -³ | -³ |
| | 5 | 68.53 | 20.09 | 26.44 | 84.35 | 110.79 | 96.79 |
| | 10 | 84.55 | 16.83 | 31.71 | 78.87 | 110.58 | 95.44 |
| | 25 | 102.42 | 8.59 | 15.37 | 33.16 | 48.53 | 56.19 |
| | 50 | 113.49 | 6.13 | 14.88 | 43.25 | 58.13 | 62.77 |

| **Metabolites** | | | | | | | |
|---|---|---|---|---|---|---|---|
| Cortisone | 1 | 104.37 | 38.94 | 66.90 | 138.17 | 205.07 | 196.46 |
| | 5 | 96.88 | 27.81 | 38.51 | 80.14 | 118.65 | 112.94 |
| | 10 | 93.67 | 24.26 | 37.06 | 80.45 | 117.51 | 108.22 |
| | 25 | 83.21 | 19.78 | 25.61 | 68.01 | 93.62 | 82.52 |
| | 50 | 84.28 | 17.20 | 18.91 | 53.54 | 72.45 | 68.23 |
| Tetrahydrocortisol | 1 | 90.23 | 35.70 | 39.35 | 88.47 | 127.82 | 113.47 |
| | 5 | 95.97 | 31.72 | 30.10 | 64.23 | 94.33 | 90.78 |
| | 10 | 103.69 | 27.61 | 27.94 | 59.57 | 87.51 | 87.88 |
| | 25 | 104.04 | 25.11 | 24.42 | 52.88 | 77.30 | 78.88 |
| | 50 | 116.79 | 17.33 | 21.16 | 59.11 | 80.27 | 80.80 |
| Tetrahydrocortisone | 1 | 103.17 | 45.30 | 50.81 | 104.79 | 155.60 | 150.09 |
| | 5 | 108.83 | 26.85 | 37.09 | 83.01 | 120.10 | 116.11 |
| | 10 | 106.80 | 25.01 | 31.64 | 70.08 | 101.72 | 99.63 |
| | 25 | 103.61 | 24.83 | 34.06 | 71.73 | 105.79 | 104.19 |
| | 50 | 110.41 | 15.60 | 21.90 | 54.21 | 76.11 | 76.61 |

| | | | | | | | |
|---|---|---|---|---|---|---|---|
| ¹ Calculation of the expanded measurement uncertainty using linear summation with a coverage factor (k) of 2 (95 % confidentiality interval) respectively of 3 (99 % confidentiality interval) ² Calculation of the expanded measurement uncertainty using quadratic summation (Nordtest method) with a coverage factor (k) of 2 (95 % confidentiality interval) ³ Insufficient respons for the qualifier ion | | | | | | | |

Since in future research matrix-matched calibration curves are not practically feasible, possible matrix effects were determined by comparing data from calibration curves in diluent to matrix-matched ones in the range from 5 µg kg⁻¹ to 50 µg kg⁻¹ for all compounds in every calcified structure.

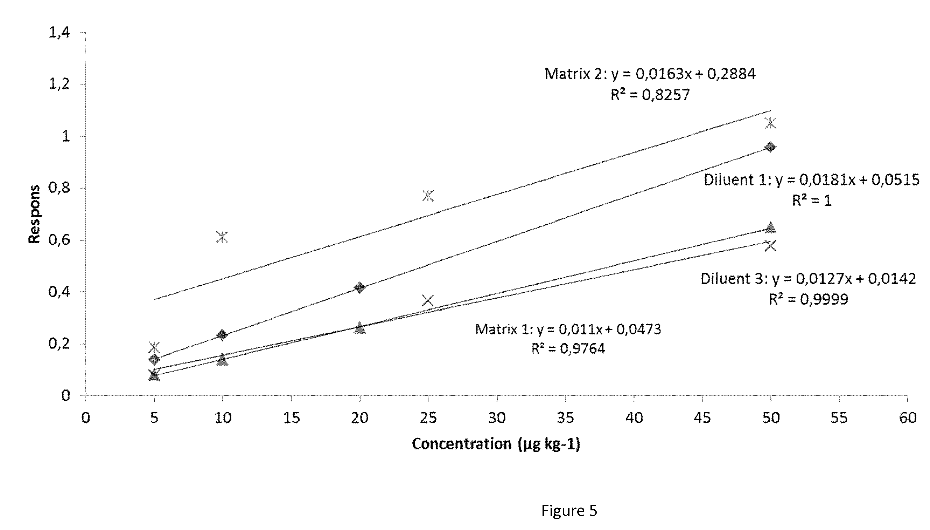

Figure 5 indicates no significant effect for cortisol in fish scales (taking into account the biological variation of the sample) but as calibration curves in diluent and matrix are normally divided, quantification using calibration curves in diluent is possible.

### Figure 5. Calibration curve in diluent and in matrix for cortisol in fish scales

Subsequently, the linearity of the response for all the compounds in every calcified structure was tested using four-fold calibration curves in diluent consisting out of five calibration points, ranging from 5 µg kg⁻¹ to 100 µg kg⁻¹, analyzed under intra-laboratory reproducibility conditions. When assessing the linearity in this range for all compounds no coefficient of determination (R²) values lower than 0.99 were found. Furthermore the calculated model indicated for all compounds normal distribution. The results for linearity, CCα, and CCβ for all compounds in a fish scale are presented in Table 3.

**Table 3: Coefficient of determination (R²) of calibration curves and values of the decision limit (CCα, µg kg⁻¹) and detection capability (CCβ, µg kg⁻¹) for all compounds in fish scales**

| Compound | R² | CCα (µg kg⁻¹) | CCβ (µg kg⁻¹) |
|---|---|---|---|
| Cortisol | 0.999 | 2.74 | 4.64 |
| Cortisone | 0.996 | 3.74 | 6.36 |
| 17α-Hydroxyprogesteron | 0.998 | 2.37 | 3.90 |
| 11-Deoxycortisol | 0.997 | 1.79 | 2.88 |
| Tetrahydrocortisol | 0.997 | 4.84 | 8.25 |
| Tetrahydrocortisone | 0.992 | 0.99 | 1.68 |

Sensitivity of the method was determined by making a dilution series on blank matrix samples resulting in a method with good sensitivity for all compounds in fish scales when taking into account the predicted working range. Selectivity of the method was proven by analyzing blank and spiked samples. In spiked samples, all compounds appeared at predicted retention times with their two ion transitions. The ion ratios were compared with the standard injection and were found acceptable according to the requirements of the Commission Decision No. 2002/657/EC. Furthermore no interfering peaks were observed.
The robustness of the method was tested during method development for all critical steps like extraction and SPE purification by varying the optimal value.
An UPLC-MS/MS method for cortisol, its precursors (17α-hydroxyprogesterone and 11-deoxycortisol), cortisone and metabolites (tetrahydrocortisol and tetrahydrocortisone) in fish scales was developed in an EN ISO/IEC 17025:2005 accredited environment and validated according the requirements of the Commission Decision No. 2002/657/EC and was proven to be suitable for its intended purposes. In addition the method was tested and validated for three other calcified structures being spine, soft fin ray and otolith. Taking into account the limitations of the latter matrices regarding sampling, sample preparation, validation results as well as their future applicability in aquaculture and wildlife, fish scales were found to be the best calcified structure for future research on chronic stress monitoring in fish.

### Example 2: Quantification of glucocorticoid levels in scales of stressed vs unstressed seabass (Dicentrarchus labrax)

### Method and materials (see example 1)

### Experimental set-up

The trial existed out of 2 replica's of 3 conditions: control (low stress, medium stress and high stress). Stress was a combination of various types of stressors (crowding, chasing and air exposure) administered ad random during a period of 3 weeks. At several unpredictable moments a day stressors were administered as indicated:
- low stress: confinement (25% volume), 30 min every 2nd day
- medium stress: confinement (25% volume), 30 min, chasing 5 min every 2nd day
- high stress: confinement (10% volume), 30 min, chasing 5 min every 2nd day, air exposure 1 min once per week

Plasma cortisol was measured in duplicate using a RIA in a 96-well plate according to Gorissen et al.⁶¹. The primary antibody shows a 100% cross reactivity with cortisol, 0.9% with 11-deoxycortisol, 0.6% with corticosterone, and < 0.01% with 11-deoxycorticosterone, progesterone, 17-hydroxyprogesterone, testosterone and estradiol. All wells except the 'non-specifics' received 100 µl cortisol antibody (Cortisol Antibody[xm210] monoclonal and IgG purified (Abcam); 1:2000 in 50 mM NaHCO3, 50 mM NaH2CO3, 0.02% NaN3, pH = 9.6) and were incubated overnight at 4°C. The following day, the plates were washed three times with 200 µl/well wash buffer (100 mM Tris, 0.9% NaCl, 0.02% NaN3). Subsequently, non-specific sites were blocked by the addition of 100 µl blocking buffer (100 mM Tris, 0.9% NaCl, 0.02% NaN3, 0.25% Normal Calf Serum) to each well. Plates were covered and incubated for one h at 37°C. Subsequently, 10 µl of standard (4 pg - 2048 pg cortisol/10 µl assay buffer containing 100 mM Tris, 0.9% NaCl, 0.1% 8-anilino-1-naphthalenesulfonic acid, 0.02% NaN3) or 10 µl of undiluted plasma or perifusion medium was added to designated wells. Non-specifics and B0 received 10 µl assay buffer. After the addition of standards and samples, 90 µl (333 Bq) of 3_{H}-hydrocortisone (PerkinElmer, USA, 1:10,000 in assay buffer) solution was added to all wells. Plates were incubated for four h at room temperature, or stored overnight at 4°C. The plates were then washed three times with wash buffer. After the final wash step, all wells received 200 µl of 'Optiphase hisafe-3 scintillation liquid' (PerkinElmer, USA) and were covered. Beta-emission was quantified by a 3 min count per well using a Microbeta Plus (Wallac/PerkinElmer, USA). Inter- and intra-assay variations were 12.5 and 3.5%, respectively.

### Results

| CORTISOL IN PLASMA | | | |
|---|---|---|---|
| | Mean cortisol concentration (ng/ml) in plasma | Standard deviation (ng/ml) | Variation coefficient (%) |
| Low stress | | | |
| Replicate 1 | 274.81 | 151.81 | 55.24 |
| Replicate 2 | 100.86 | 101.70 | 100.84 |
| All replicates (20 samples) | 183.26 | 152.90 | 83.43 |

| Medium stress | | | |
|---|---|---|---|
| Replicate 1 | 319.00 | 106.04 | 33.24 |
| Replicate 2 | 190.58 | 136.65 | 71.70 |
| All replicates (20 samples) | 254.79 | 136.06 | 53.40 |

| High stress | | | |
|---|---|---|---|
| Replicate 1 | 112.64 | 95.22 | 84.53 |
| Replicate 2 | 187.92 | 73.90 | 39.32 |
| All replicates (20 samples) | 150.28 | 91.50 | 60.89 |

| CORTISOL IN SCALE | | | |
|---|---|---|---|
| | Mean cortisol concentration (µg kg⁻¹) in scale | Standard deviation (µg kg⁻¹) | Variation coefficient (%) |
| Low stress | | | |
| Replicate 1 | 1.90 | 1.00 | 53.58 |
| Replicate 2 | 1.50 | 1.70 | 109.19 |
| All replicates (20 samples) | 1.70 | 1.35 | 79.58 |

| Medium stress | | | |
|---|---|---|---|
| Replicate 1 | 2.40 | 2.00 | 83.29 |
| Replicate 2 | 2.10 | 1.50 | 69.13 |
| All replicates | 2.27 | 1.72 | 75.86 |

| High stress | | | |
|---|---|---|---|
| Replicate 1 | 3.20 | 2.30 | 69.57 |
| Replicate 2 | 3.60 | 4.00 | 109.08 |
| All replicates (20 samples) | 3.44 | 3.15 | 91.50 |

### Example 3: Quantification of glucocorticoid levels in scales of 'zebrafish (Danio rerio), carp (Cyprinus carpio), and tilapia (Oreochromis mossambicus)

### Method and materials (see example 1)

### Experimental set-up

The trial with zebrafish, carp and tilapia exists out of 2 replica's of 4 conditions, each with 6 fish: control (unstressed), dexamethasone (inhibition of stress pathway), cortisol (activation of stress pathway) and stress induced by various stressors given in an ad random administered manner for 6 weeks.

### Example 4: Quantification of glucocorticoid levels in scales of 'seabass (Dicentrarchus labrax)and salmon (Salmo salar)

### Method and materials (see example 1)

### Experimental set-up

The trial with seabass and salmon exists out of 2 replica's of 4 conditions, each with 6 fish: control (unstressed), dexamethasone (inhibition of stress pathway), cortisol (activation of stress pathway) and stress induced by various stressors given in an ad random administered manner for 6 weeks.

### References

(1) Sharpe, C. S. Prog. Fish-Cult. 1998, 60, 81-87.
(2) Wendelaar Bonga, S. E. Physiol. Rev. 1997, 77, 591-626.
(3) Barton, B. A. Integr. Comp. Biol. 2002, 42, 517-525.
(4) Fairhurst, G. D; Marchant , T. A; Soos, C.; Machin, K. L.; Clark, R. G J. Exp. Biol. 2013, 216, 4071-4081.
(5) Vachon, P.; Moreau, J. P. J. Am. Assoc. Lab. Anim. Sci. 2001, 40, 22-24.
(6) Sapolsky, R.M., Romero L. M., Munck, A. U. Endocr. Rev. 2000, 21, 55-89.
(7) Blas, J.; Bortolotti, G. R.; Tella, J. L.; Baos, R.; Marchant, T. A. Proc. Natl. Acad. Sci. U. S. A. 2007, 104, 8880-8884.
(8) McEwen, B. S.; Wingfield, J. C. Horm. and Behav. 2003, 43, 2-15.
(9) Korte, S. M.; Koolhaas, J. M.; Wingfield, J. C. McEwen, B. S. Neurosci. Biobehav. Rev. 2005, 29, 3-38.
(10) Øverli, Ø.; Sørensen, C.; Pulman, K. G. T.; Pottinger, T. G.; Korzan, W.; Summers, C. H.; Nilsson, G. E. Neurosci. Biobehav. Rev. 2007, 31, 396-412.
(11) Easy. R. H.; Ross, N. W. J. Fish Biol. 2010, 77, 1616-1631.
(12) Kammerer, B. D.; Cech, J. J., Kultz, D. Comp. Biochem. Physiol., Part A: Mol. Integr. Physiol. 2010, 157, 260-265.
(13) Small, B. C., Aquaculture 2003, 218, 177-185.
(14) Pramod, P. K.; Ramachandran, A.; Sajeevan, T. P.; Thampy, S.; Pai, S. S. Aquacult. Res. 2010, 41, 309-314.
(15) Palić, D.; Herolt, D. M.; Andreasen, C. B.; Menzel, B. W.; Roth, J. A. Aquaculture 2006, 254, 675-685.
(16) Ruane, N. M.; Komen, H. Aquaculture 2003, 218, 685-693.
(17) Ellis, T.; James, J. D.; Stewart, C.; Scott, A. P. J. Fish Biol. 2004, 65, 1233-1252.
(18) Turner, J. W. Jr.; Nemeth, R.; Rogers, C. Gen. Comp. Endocrinol. 2003, 133, 341-352.
(19) Lupica S. J.; Turner J. W. Jr. Aquacult. Res. 2009, 40, 437-441.
(20) Cook, N. J. Can. J. Anim. Sci. 2012, 92, 227-259.
(21) Bertotto D., Poltronieri C., Negrato E., Majolini D., Radaelli G., Simontacchi C. Aquacult. Res. 2010, 41, 1261-1267.
(22) Payan P.; De Pontual, H.; Edeyer,A.; Borelli, G.; Boeuf, G.; Mayer-Gostan, N. Can. J. Fish. Aquat. Sci. 2004, 61, 1247-1255.
(23) Casselman, J. M. Trans. Am. Fish. Soc. 1990, 119, 673-688.
(24) Francis, R. I. C. C. J. Fish Biol. 1990, 36, 883-902.
(25) Ricker, W. E. Can. J. Fish. Aquat. Sci.1992, 49, 1018-1026.
(26) Gauldie, R.W., Nelson, D. G. A. Comp. Biochem. Physiol., Part A: Mol. Integr. Physiol. 1988, 90, 501-509.
(27) Carlström, D., Biol. Bull. 1963, 125, 441-463.
(28) Bury N. R., Sturm A. Gen. Comp. Endocrinol. 2007, 153, 47-56.
(29) http://www.chemspider.com/Chemical-Structure.5932.html
(30) Cirillo N., Prime S. S. J. Cell. Biochem.2011 112, 1499-1505.
(31) Raul J-S., Vincent Cirimele V., Bertrand Ludes B., Pascal Kintz P. Clin. Biochem. 2004, 37, 1105-1111.
(32) Gow R., Thomson S., Rieder M., Van Uum S., Koren G. Forensic Sci. Int. 2010, 196, 32-37.
(33) Sugawara E. K., Verreschi L. Quim. Nova 2010, 33, 447-450.
(34) Yajie Xiao Y., Chan S. W., Hu M., Chu T. T. W., Fok B. S. P., Poon E. W. M., Tomlinson B Chromatographia 2012, 75, 169-173
(35) Gao W, Xie Q, Jin J., Qiao T., Wang H., Chen L., Deng H., Lu Z Clin. Biochem. 2010, 43, 677-682.
(36) Shibata N., Hayakawa T., Takada K., Hoshino N., Minouchi T., Yamaji A. J. Chromatogr B 1998, 706, 191-199.
(37) Shibasakia H., Nakayamaa H., Furutaa T., Kasuyaa Y., Tsuchiyab M., Soejimab A., Yamadab, Nagasawab T. J. Chromatogr. B 2008, 870, 164-169.
(38) Ahmadkhaniha R., Shafiee A., Rastkari N., Khoshayand M. R., Kobarfard F. J. Chromatogr B 2010, 878, 845-852.
(39) O'Connor E. A., Pottinger T. G., Sneddon L. U. Fish Physiol. Biochem. 2011, 37, 461-469.
(40) Benjamín Costas B., Conceição L. E. C., , Aragão C., Martos J. A., Ruiz-Jarabo I. Mancera J. M., Afonso A. Aquaculture 2011, 316, 68-76.
(41) Tellis M. S., Alsop D., Wood C. M., Comp. Bioch. Fysiol. 2012, 155, 281-289.
(42) Toorchi M., Bani A., Alizadehsabet H. J. Appl. Ichthyol. 2012, 28, 130-134
(43) Sun K., Ramgir N., Bhansali S. Sens. Actuators, B 2008, 133, 533-537.
(44) Mitchell J. S., Lowe T. E., Ingram J. R. Analyst 2009, 134, 380-386
(45) Commission Decision No. 2002/657/EC, Concerning the performance of analytical methods and the interpretation of results. Off. J. Eur. Commun. 2002*.*
(46) EN ISO/IEC 17025:2005, General requirements for the competence of testing and calibration laboratories. CEN/CENELEC, 2005*.*
(47) ISO GUM, Guide to the Expression of Uncertainty in Measurement (GUM); 1st edition; NBN-X-40-001, 1995.
(48) Eurachem/CITAC Guide, Quantifying Uncertainty in Analytical Measurement (QUAM); 2nd ed; http://www.eurachem.org/, 2000**.**
(49) Royal Decree of the Belgian State of January 18th 2012 published in the Belgian Government Gazette of January 27th 2012, 2012.
(50) Eurolab Technical Report No. 1/2007; Measurement uncertainty revised: Alternative approaches to uncertainty evaluation.; http://www.eurotab.org/, 2007**.**
(51) ISO/TS 21748:2004, Guidance for the use of repeatability, reproducibility and trueness estimates in measurement uncertainty estimation., 2004**.**
(52) Nordtest Technical Report TR 537; Handbook for calculation of measurement uncertainty in environmental laboratories.; 2nd ed (approved 2004-02); http://www.nordtest.org/, 2004**.**
(53) Ellis, T.; Sanders, M. B.; Scott, A. P. Wiener Tierärztliche Monatsschrift 2013, 100, 255-269.
(54) McWhinney B.C., Ward G., Hickman P.E. Clin. Chem. 1996, 42 (6), 979-981.
(55) Flor, S. Lucangioli S., Contin M., Tripodi V. Electrophoresis 2010, 31, 3305-3313.
(56) Heistermann M., Palme R., Ganswindt A. Am. J. Primatol. 2006, 68, 257-273.
(57) Cirimele V., Kintz P., Dumestre V., Goullé J.P., Ludes B. Forensic Sci. Int. 2000, 107, 381-388.
(58) Raul J-S., Cirimele V., Ludes B., Kintz P. Clin. Biochem. 2004, 37, 1105-1111.
(59) ISO/DIS 5725:1994, Accuracy of Measurement Methods and Results., 1994**.**
(60) Barnett V.; Lewis T. Outliers in Statistical Data; 3rd ed., 1994**.**
(61) Gorissen M., Bernier N. J., Manuel R., de Gelder S., Metz J. R., Huising M. O., Flik G. Gen. Comp. Endocrinol. 2012, 178, 75-81. (

## Claims

1. An in vitro method to quantify the level of chronic stress in a fish comprising:
- obtaining at least one scale from said fish,
- extracting glucocorticoids from said scale,
- purifying the glucocorticoids extracted from said scale,
- quantifying said purified glucocorticoids, and
- correlating said quantified glucocorticoids with a level of chronic stress.

2. A method according to claim 1 glucocorticoids are cortisol, corticosterone, 17α-hydroxyprogesterone, 11-deoxycortisol, 11-deoxycorticosterone, cortisone, tetrahydrocortisol and/or tetrahydrocortisone.

3. A method according to claims 1-2, wherein said fish belong to the infraclass of Teleostei.

4. A method according to claims 1-3, wherein said obtaining at least one scale from said fish is obtaining between 40 and 80 scales from said fish.

5. A method according to claims 1-4, wherein said extracting glucocorticoids is undertaken by adding methanol as extraction solvent.

6. A method according to claim 5, wherein said extraction with methanol is followed by a solid phase extraction.

7. A method according to claims 1-6, wherein said quantifying of extracted glucocorticoids is undertaken by liquid chromatography coupled to tandem mass spectrometry, high performance liquid chromatography coupled to ultraviolet, diode array or fluorescence detection, gas chromatography, a radio immunoassay, an enzymatic immunoassay or a sensor based technique.

8. A method according to claim 7, wherein said liquid chromatography coupled to tandem mass spectrometry is ultra-performance liquid tandem mass spectrometry.

9. A method according to claim 7, wherein said immunoassay is a radio immunoassay or an enzyme immunoassay.

10. Use of fish scales sampled from a fish to quantify the level of chronic stress of said fish.

11. Use according to claim 10 wherein said quantification is undertaken by a method according to claims 1-9.
